# EUROPEAN PATENT APPLICATION

(11) **EP 1 672 365 A1**
(43) Date of publication of application: **21.06.2006**
(21) Application number: 04078414.2
(22) Date of filing: 16.12.2004
(51) Int. Cl.: G01N 33/50, G01N 33/68, A01K 67/027

(54) **Means and methods for determining and/or influencing cellular transportation**

(71) Applicant: Het Nederlands Kanker Instituut, 1066 CX Amsterdam (NL)
(72) Inventor: Jonker, Johan Willem, 1066 TX Amsterdam (NL); van Herwaarden, Antonius Eduard, 1058 EC Amsterdam (NL); Schinkel, Alfred Hermanus, 1052 WJ Amsterdam (NL)
(74) Representative: Winckels, Johannes Hubertus F.

(57) **Abstract**

The invention provides a method for determining whether a compound or its metabolite can be secreted into the milk or colostrum of a lactating mammal through active transport by Breast Cancer resistance Protein if said compound or metabolite is circulating within said mammal, the method comprising determining whether said compound or metabolite is a substrate of Breast Cancer Resistance Protein. Said compound preferably comprises a nutrient, a medicament, a vaccine, a pesticide, a toxin and/or a carcinogen. A method for altering the capability of a compound of being secreted into the milk of a lactating mammal if said compound is circulating within said mammal, the method comprising altering at least part of said compound such that said compound's capability of being a substrate of Breast Cancer Resistance Protein is altered, is also provided.

## Description

The invention relates to the field of biology. More specifically, the invention relates to transportation pathways.

Milk is the sole food for young mammals and an important constituent of the human diet. However, in addition to nutrients also many adverse compounds such as drugs, carcinogens and environmental toxins are capable of accumulating in milk via poorly understood pathways, posing a health risk to breast-fed infants, young suckling non-human mammals and consumers of dairy products in general. It is therefore generally advised to avoid smoking during breast-feeding and to be careful with use of medication during breast-feeding and dairy milk production. It is however not always possible to avoid the intake of compounds involving a health risk to young mammals and/or milk consuming individuals. For instance, the use of medication is sometimes unavoidable. Breast feeding humans and/or lactating non-human mammals suffering from (infectious) disease often need to be treated in order to prevent the disease to become more severe. This results in a health risk for breast-fed infants/ young suckling mammals, and/or in spoilage of milk of dairy mammals since the milk of a mammal receiving medication is often not allowed to be used for consumption.

Another risk involved with transportation of compounds into the milk of a lactating mammal is the long-term exposure to common environmental and dietary toxins through milk. The dietary carcinogen and toxin 2-amino-1-methyl-6-phenylimidazo[4,5-b]pyridine (PhIP) for example, which is abundantly present in well-done meat and cigarette smoke, is actively concentrated into milk. PhIP is a powerful mutagen that induces intestinal tumors in rodent neonates through milk exposure and it has been implicated in human breast carcinogenesis.

In view of the health problems and economic problems involved with adverse compounds such as medicaments and pesticides accumulating in milk, it is preferred to use compounds which do not accumulate in milk. However, such compounds are often not available. Since the mechanism of transportation into the milk of a lactating mammal was not understood before the present invention, it was not possible to design compounds such as medicaments, vaccines and pesticides which do not accumulate in milk. When new medicaments are developed, it is not known beforehand whether they will accumulate into the milk of lactating mammals. No in *vitro* test is available for determining whether a given compound is capable of being transported into milk. Therefore, each medicament has to be tested in *vivo* after it has been produced. A newly developed medicament is for instance administered to a lactating non-human animal. If such new medicament appears to be transported into the milk of said animal, it is contra-indicated for lactating mammals. Hence, specifically designing a medicament which is not capable of being transported into milk was not possible before the present invention. It was rather a matter of trial and error.

On the other hand, compounds that are beneficial to breast-fed infants or young suckling non-human mammals, such as for instance certain nutrients and/or medicaments which would benefit the young mammal, are not always accumulated in milk and have to be provided otherwise. Before the present invention it was not known how such beneficial compounds could be selected or altered in order to provide for the property of being transported into milk.

In view of the above mentioned problems and limitations involved with lactating mammals, it is desirable to be capable of determining whether a compound or its metabolite is at risk of accumulating in milk and/or colostrum of a lactating mammal if said compound or metabolite is circulating within said lactating mammal. Furthermore, it is advantageous to be capable of influencing a capability of a certain compound or metabolite of accumulating in milk and/or colostrum, such that beneficial compounds accumulate in milk whereas adverse compounds do not. However, before the present invention, it was not known by what pathway(s) compounds are capable of accumulating in milk and/or colostrum.

It is an object of the present invention to provide means and methods for determining and/or influencing a capability of a compound of being transported across a cell membrane. It is a preferred object to determine and/or influence a capability of a compound of accumulating in milk and/or colostrum of a lactating mammal.

The invention provides a method for determining whether a compound or its metabolite can be transported through active transport by Breast Cancer Resistance Protein into the milk or colostrum of a lactating mammal if said compound or metabolite is circulating within said mammal, the method comprising determining whether said compound or metabolite is a substrate of Breast Cancer Resistance Protein.

The invention provides the insight that Breast Cancer Resistance Protein is involved with transport of compounds into the milk or colostrum of a lactating mammal. The Breast Cancer Resistance Protein (BCRP/ABCG2) is an ATP binding cassette (ABC) transmembrane drug transporter. BCRP is a protein of about 72 kDa, localized in the plasma membrane, capable of conferring multidrug resistance to tumor cells. BCRP actively extrudes a wide variety of drugs, carcinogens and dietary toxins from cells. Because of its apical localization in epithelia of the intestine, kidney and placenta and in the bile canalicular membrane, it reduces systemic, tissue and fetal uptake of these xenotoxins, and mediates their extrusion from the body. BCRP is described in more detail in Allen et al. Molecular Cancer Therapeutics (2002) Vol. 1, 427-434. It is to be understood that the term "BCRP" comprises any corresponding gene or expression product thereof in other organisms. The involvement of BCRP with transportation of compounds into milk or colostrum of a lactating mammal was not known before the present invention.

According to the present invention BCRP is present in the apical membrane of alveolar mammary gland epithelial cells of mammals during late pregnancy and during lactation. BCRP is capable of transporting a substrate into the milk or colostrum of a lactating mammal. Hence, a compound or its metabolite which is a substrate for BCRP has an increased chance of being secreted into the milk or colostrum of a lactating mammal if said compound or metabolite is at least in part circulating within said mammal, as compared to a compound which is not a BCRP substrate. The extent of in *vivo* transportation of a BCRP substrate depends on the extent of interaction between said substrate and BCRP located in mammary gland epithelial cells. A method of the invention is therefore preferably performed in order to test a compound or metabolite that is capable of circulating in the blood circulation of said mammal, so that said compound or metabolite is capable of being readily contacted with mammary gland epithelial cells. Moreover, a method of the invention is preferably performed in order to test a compound or metabolite that is not capable of being bound by another component of an animal's body to such extent that transportation by BCRP is essentially inhibited.

If a BCRP substrate, or a compound whose metabolite is a BCRP substrate, is administered to said lactating mammal, resulting in circulation of said BCRP substrate within said animal, it is at risk of being transported by BCRP into the milk or colostrum of said animal. Hence, compounds such as for instance medicaments, pesticides, environmental toxins or carcinogens that are taken up by a lactating mammal and that are a substrate for BCRP are at risk of being transported into the milk or colostrum of said lactating mammal. Moreover, metabolites of such compounds which metabolites are BCRP substrates are at risk of being transported into the milk or colostrum of said lactating animal. Therefore, if transport into milk is to be avoided, it is preferred to select a compound and/or metabolite which is not a BCRP substrate. Alternatively, a BCRP substrate is modified such that its capability of being transported by BCRP is lost.

The insight of the present invention is used for determining whether a candidate compound or its metabolite is at risk of being transported into milk through active transport by BCRP. In one embodiment a screening procedure is performed. It is for instance tested whether a compound that is harmful for a young mammal or for milk consuming individuals is a BCRP substrate. This test is easily performed, preferably in *vitro,* as outlined below. If said compound appears to be a BCRP substrate, it is preferred to select another compound, or to modify said compound, because a BCRP substrate is at risk of being transported into milk through active transport by BCRP. Hence, a compound which is a BCRP substrate is at risk of accumulating in the milk of a lactating animal. Therefore, during production of a compound such as a medicament, pesticide or a vaccine which is harmful for young mammals and/or milk consuming individuals, a compound which is not a BCRP substrate is preferably used. One embodiment of the invention therefore provides a method for determining whether a compound or its metabolite can be transported through active transport by Breast Cancer Resistance Protein into the milk or colostrum of a lactating mammal if said compound or metabolite is circulating within said mammal, the method comprising determining whether said compound or metabolite is a substrate of Breast Cancer Resistance Protein and, if said compound appears to be a BCRP substrate, typing said compound as a candidate for secretion into the milk or colostrum of a lactating animal through active transport by BCRP when said compound is provided at the basolateral side of said animal's mammary gland epithelial cells. If said compound appears not to be a BCRP substrate, it is typed as not being a candidate for secretion into the milk or colostrum of a lactating animal through active transport by BCRP.

By a substrate of BCRP is meant herein a compound which is capable of being specifically bound by BCRP and transported across a cell membrane, preferably into the milk or colostrum of a lactating mammal. Said substrate is preferably transported by BCRP from a breast epithelial cell layer into milk.

A compound of the present invention comprises any kind of compound which is capable of being administered to a lactating animal. A compound of the present invention preferably comprises an organic molecule of about 50-3000 Dalton. More preferably said compound comprises a compound of about 150-2000 Dalton, most preferably a compound of about 200-1000 Dalton because BCRP is particularly capable of transporting organic compounds of these sizes. In one embodiment said compound comprises a peptide. Said peptide preferably comprises about 3-20 amino acid residues. More preferably said peptide comprises about 5-15 amino acid residues.

A metabolite of a compound is defined as a molecule which is formed when said compound is processed in *vivo*. After administration of a compound such as for instance a prodrug to an animal, said compound is sometimes altered within said animal. Said compound is for instance cleaved. As another example said compound, or a metabolite thereof, is modified by conjugation with an endogenous molecule such as for instance glucuronic acid, glutathione and/or sulfate. A metabolite resulting from such modification may subsequently be cleaved, and/or a cleavage product may subsequently be modified. Any product resulting from in *vivo* processing of a compound is called herein a metabolite of said compound.

Active transport by BCRP means transportation of a BCRP substrate across a membrane. Said BCRP substrate and BCRP specifically bind, after which BCRP transports this substrate from one side of a cell, or a membrane, to another side of said cell or membrane. Preferably, said substrate is transported from a basolateral side of an epithelial cell to an apical side of said cell.

A lactating mammal is defined herein as a mammal that produces milk or colostrum, or a mammal that starts producing milk or colostrum while said compound or a metabolite thereof is circulating within said mammal. Hence, a method of the invention is suitable for determining whether a compound or its metabolite will be secreted into the milk or colostrum of a mammal upon administration of said compound to a mammal that lactates or starts lactating after said compound has been administered, and while at least part of said compound or metabolite is still circulating within said mammal.

A mammal of the invention preferably comprises a human individual or a dairy animal. A mammal of the invention for instance comprises a female mammal during late pregnancy and/or a female mammal who is lactating after birth of her baby. A mammal of the invention also comprises a mammal without a baby which mammal is nevertheless lactating, such as a dairy animal that continues lactating. Said dairy mammal preferably comprises a cow, a sheep, a goat, a yak, a camel, a llama, a mare or a rabbit, since these mammals produce milk that is suitable for further use by humans.

Colostrum is a milk-like substance produced by a lactating mammal in the early days of lactating. This special milk is often high in carbohydrates, protein and antibodies.

The basolateral side of an epithelial cell is defined as the side of said cell that is normally adjacent to the extracellular matrix *in vivo*. If said epithelial cell is used in *vitr*o it is often still possible to determine what side is the basolateral side, because epithelial cells are polarized. For instance, the nucleus is often present in the basolateral half of the cell. The apical side of an epithelial cell is defined as the side of said cell which is normally exposed to a compartment of an animal such as the alveolar lumen which is considered the exterior. Hence, the basolateral side of epithelial cells lining an alveolar lumen of a mammary gland is directly or indirectly exposed to the blood stream, while the apical side of said epithelial cells is exposed to the alveolar lumen.

A functional part of a Breast Cancer Resistance Protein is defined as a part which has the same transportation properties in kind, not necessarily in amount. By transportation properties is meant the capability to transport a BCRP substrate across a cell membrane. A functional part is for instance produced by deleting at least one amino acid residue of BCRP such that a transportation capability - in kind, not necessarily in amount - is maintained. A derivative of a BCRP is defined as a proteinaceous molecule which has been altered such that the transportation properties of said molecule are essentially the same in kind, not necessarily in amount. A derivative is provided in many ways, for instance through conservative amino acid substitution. Conservative substitution comprises a substitution of one amino acid residue with another residue with generally similar properties (size, hydrophobicity, etc), such that the overall functioning is not seriously affected.

A person skilled in the art is well able to generate analogues of a BCRP. This is for instance done through screening of a peptide library. Such an analogue has essentially the same immunogenic properties of said protein in kind, not necessarily in amount. In one embodiment said analogue comprises non natural residues, such as D-amino acid residues and/or modified amino acid residues.

By at least a functional part of a BCRP gene is meant a part of said gene, at least 30 base pairs long, preferably at least 200 base pairs long, comprising at least one expression characteristic (in kind not necessarily in amount) as BCRP. Preferably said part encodes a proteinaceous molecule capable of at least in part transporting a BCRP substrate. An analogue of a BCRP gene is defined herein as a molecule with at least one characteristic (in kind, not necessarily in amount) as a BCRP gene. Said analogue for instance comprises peptide nucleic acid (PNA). A functional part or an analogue of a BCRP gene preferably encodes BCRP, or a functional part, derivative and/or analogue thereof.

A compound is circulating in a mammal if said compound is spreading within at least part of said mammal such that different kinds of locations within said mammal are reached. Said compound is preferably circulated within the blood circulation of said mammal. While circulating, said compound reaches a mammary gland epithelial cell layer comprising BCRP, and/or is transported into said layer. If a compound that is a BCRP substrate is contacted with BCRP of a mammary gland it is actively transported by BCRP into the milk or colostrum of a lactating mammal. In one preferred embodiment, said compound's BCRP binding properties remain essentially the same - in kind, not necessarily in amount - during circulation *in vivo,* so that the result of an in vitro assay determining whether said compound is a BCRP substrate remains indicative for said compound circulating in *vivo. In vivo* modifications, or *in vivo* binding of said compound to another molecule, resulting in diminished or lost BCRP specificity is preferably avoided in case transport of a beneficial compound into milk is desired. In another preferred embodiment it is determined into what metabolite(s) a given compound is converted *in vivo*. For instance, a prodrug is designed that is converted in vivo into an active medicament. Once it is known into what metabolite(s) a compound is converted in *vivo,* said metabolite(s) is/are preferably tested with a method of the invention in order to determine whether said metabolite(s) is/are a BCRP substrate. If a metabolite appears to be a BCRP substrate, it is at risk of being secreted into the milk or colostrum of a lactating mammal once said metabolite is circulating within said mammal.

In one embodiment a compound or its metabolite is circulating within said mammal as a result of administration of said compound to said mammal. It is possible to administer a compound to a mammal in various ways. Said compound is for instance administered enterally, parenterally, intrapulmonary and/or via dermal application.

Many different kind of methods are available in the art for determining whether a compound is a substrate of BCRP.

In one embodiment an *in* vitro inside-out vesicle uptake experiment is used. In this embodiment a vesicle comprising BCRP, or a functional part, derivative and/or analogue thereof, is used which is oriented such that a BCRP substrate is transported into the vesicle. After incubation with at least one candidate compound it is determined whether said candidate compound is accumulating in the vesicle, indicating that it has been transported into the vesicle by said BCRP or functional part, derivative and/or analogue, which in turn indicates that said compound is a BCRP substrate. In one embodiment a control is used in order to confirm that said candidate compound is accumulating in said vesicle in a BCRP-dependent manner. BCRP-dependence is preferably established by comparison with an analogous inside-out vesicle not comprising BCRP or a functional part, derivative and/or analogue thereof. This assay is particularly suitable for testing hydrophilic compounds since hydrophilic compounds essentially do not diffuse into/through a hydrophobic vesicle membrane.

In another embodiment, a transwell experiment is performed for determining whether a compound is a substrate of Breast Cancer Resistance Protein. In this embodiment basolateral to apical transport by BCRP is determined as follows. A transwell membrane, preferably a polycarbonate filter, is incubated with BCRP comprising cells, or cells comprising a functional part, derivative and/or analogue of BCRP, preferably epithelial cells. The cells are allowed to grow, preferably into a confluent layer. The cell coated membrane is placed into a well such that the cells separate two compartments, preferably an upper (apical) and lower (basolateral) compartment. A candidate compound is added, for instance to the basolateral side of the cells. After incubation it is determined whether said candidate compound is transported to the apical side of the cells. If said candidate compound appears to be present at the apical side of the cells, at least to a larger extent than is observed when cells without BCRP or functional part, derivative or analogue thereof are used, it indicates that said compound is a BCRP substrate.

In one embodiment a candidate compound is added to the apical side of said cells. If passive transport of said candidate compound to the basolateral side of said cells occurs to some extent, said compound will be present at the basolateral side after a while. If said compound is a BCRP substrate, it will be, at least in part, transported back by BCRP to the apical side. Hence, when cells comprising BCRP or a functional part, derivative and/or analogue thereof are used, and when passive transport occurs to some extent, a BCRP substrate will be present at the basolateral side to a lower extent as compared to an experiment wherein cells are used that do not comprise BCRP or a functional part, derivative and/or analogue thereof. Moreover, when cells comprising BCRP or a functional part, derivative and/or analogue thereof are used, and when passive transport occurs to some extent, a BCRP substrate will be present at the basolateral side to a lower extent as compared to a compound which is not a BCRP substrate. Thus, when a first compound is added to the apical side of the cells, and when said first compound appears at the basolateral side of the cells to a lower extent than is observed when cells without BCRP or functional part, derivative or analogue thereof are used, it indicates that said first compound is a BCRP substrate.

A transwell experiment is particularly suitable for testing hydrophobic compounds because hydrophobic compounds are capable of entering an epithelial cell layer. If a hydrophilic compound is tested it is preferred to use a transport system capable of transporting said hydrophilic compound into the epithelial cell layer. More preferably, however, a hydrophilic compound is tested with an inside-out vesicle uptake experiment.

In yet another embodiment transport of a candidate compound by cells comprising BCRP, or a functional part, derivative and/or analogue thereof, preferably compared with cells not comprising BCRP or a functional part, derivative or analogue thereof, is determined. Preferably, said cells comprising BCRP or a functional part, derivative and/or analogue thereof and said cells not comprising BCRP or functional part, derivative or analogue thereof are the same kind of cells, such as for instance epithelial cells. In this embodiment it is determined whether cells comprising BCRP or a functional part, derivative and/or analogue thereof are capable of transporting a candidate compound and whether cells essentially not comprising BCRP or a functional part, derivative or analogue thereof are less, or essentially not, capable of transporting said candidate compound. If this is the case, it indicates that said candidate compound is a BCRP substrate. A reduced accumulation of a candidate compound inside cells comprising BCRP, or a functional part, derivative and/or analogue thereof, as compared to the accumulation of said candidate compound inside cells not comprising BCRP or a functional part, derivative and/or analogue thereof, is also a good indication that said compound is a BCRP substrate. A reduced accumulation inside cells comprising BCRP, or a functional part, derivative and/or analogue thereof, indicates that said compound is transported out of the cell by BCRP, or by said functional part, derivative and/or analogue thereof.

In yet another embodiment it is determined whether an inhibitor of BCRP is capable of influencing transport of a candidate compound by cells comprising BCRP, or a functional part, derivative and/or analogue thereof. Cells comprising BCRP or a functional part, derivative and/or analogue thereof are incubated with a candidate compound, either with or without the presence of a BCRP inhibitor. If a candidate compound is transported by said cells in the absence of a BCRP inhibitor, and if said candidate compound is less, or essentially not, transported by said cell in the presence of said inhibitor, it indicates that said candidate compound is transported by BCRP or a functional part, derivative and/or analogue thereof and, hence, that said compound is a substrate of BCRP. Many BCRP inhibitors are known in the art, such as for instance Fumitremorgin C, Ko143, Ko134, (described in Allen et al., Molecular Cancer Therapeutics, Vol 1, 417-425, April 2002) GF120918 (*N*-{4-[2-(1,2,3,4-tetrahydro-6,7-dimethoxy-2-isoquinolinyl)-ethyl]-phenyl}-9,10-dihydro-5-methoxy-9-oxo-4-acridine carboxamide, described in Hyafil F et al. (1993) Cancer Res.53:4595-4602), CI1033, Iressa, novobiocin, flavopiridol, reserpine and XR9576. Preferably, inhibitor GF120918 is used.

It is also possible to test in *vivo* whether a compound is a substrate of BCRP, using a non-human animal. In one embodiment it is determined whether a candidate compound or its metabolite is transported into the milk of a lactating non-human animal expressing BCRP in its mammary gland cells. Said compound or metabolite is administered to said non-human animal. If said compound or metabolite appears to be transported into the milk of said non-human animal, it suggests that said compound or metabolite is a BCRP substrate. Preferably, as an extra control, said candidate compound is also administered to a non-human animal that does not, or to a lesser extent, express BCRP in its mammary gland cells. If less compound accumulates in the milk of said non-human animal as compared to the amount of compound that accumulates in the milk of an animal expressing BCRP, it indicates that said compound is a BCRP substrate.

Since transportation properties of BCRP derived from a non-human animal often differ somewhat from transportation properties of human BCRP, it is preferred to use a non-human animal provided with human BCRP or a functional part, derivative and/or analogue thereof. As used herein, the term human BCRP includes human BCRP or a functional part, derivative and/or analogue thereof. Said human BCRP is preferably present in the apical membrane of alveolar mammary gland epithelial cells of said non-human animal. Preferably a transgenic non-human animal expressing human BCRP in its mammary glands is used. In one embodiment said non-human animal comprising human BCRP is provided with a candidate compound. If said candidate compound or at least one metabolite thereof appears to be transported into the milk of said transgenic animal, it suggests that said compound or metabolite is a substrate of human BCRP. Preferably it is determined whether said candidate compound or metabolite is transported into the milk of said transgenic animal to a larger extent than in a non-transgenic animal of the same species. This way, more insight is gained into the chance of accumulation of said compound and/or metabolite in human milk upon administration to a human individual.

If an animal which normally expresses endogenous BCRP in its mammary glands, such as a mouse, is provided with human BCRP it is preferred to diminish expression of endogenous BCRP. More preferably, expression of endogenous BCRP is essentially inhibited. Accumulation of a compound in the milk of a non-human animal which is expressing human BCRP and which does not, or to a significantly lesser extent, express endogenous BCRP, indicates that said compound is a substrate of human BCRP. Hence, it is more accurately determined whether said compound is suitable for administration to a human individual. Expression of endogenous BCRP is diminished or inhibited in various ways known in the art. For instance, said endogenous BCRP gene is at least in part deleted and/or substituted via homologous recombination. Preferably, endogenous BCRP is exchanged for human BCRP. In another embodiment an endogenous BCRP gene is rendered inactive, for instance via site directed mutagenesis resulting in an inactive gene or by administration of a nucleic acid molecule comprising an antisense sequence such as for instance small interfering RNA (siRNA).

In one embodiment a BCRP knockout mutant, preferably a BCRP knockout mouse, is used. A BCRP knockout mutant is an animal that has essentially lost its ability of expressing endogenous BCRP in its mammary glands. A BCRP knockout mutant is produced in various ways known in the art, such as for instance via homologous recombination, site directed mutagenesis or by administration of an antisense nucleic acid or small interfering RNA, as outlined above.

In a preferred embodiment said compound is administered to said non-human animal in the same way as it is normally acquired by a mammal. Hence, a medicament that is normally administered orally to a patient is preferably also orally administered to a non-human animal in a method of the present invention. Of course, in order to determine whether said compound is secreted into the milk or colostrum of a lactating non human animal, it is preferred to take care that said compound is not administered to the milk or colostrum of said animal because in that case the presence of said compound in the milk is not the only result *of in vivo* transportation. In a preferred embodiment said compound or metabolite is administered orally, intrapulmonary, via dermal application or via intracutaneous, subcutaneous or intramuscular injection.

Many more alternative methods are available in the art for determining whether a candidate compound is a substrate of BCRP. These alternative methods are also suitable for performing a method of the present invention. Preferably, a method of the invention comprises determining in vitro whether a compound or metabolite is a substrate of BCRP, because an in vitro test avoids the risk of suffering of non-human animals and it is often cheaper. An *in vitro* test is therefore preferably performed during early stages of investigation. In one embodiment an *in vitro* test is performed in order to select one, or several, promising candidate compounds, after which an in *vivo* test is performed with at least one selected compound in order to study its in vivo characteristics in more detail.

A method of the invention is suitable for testing a wide variety of compounds. If a compound is at risk of accumulating in milk, its administration is sometimes undesired, for instance in case of administration of a pesticide, medicament or vaccine. At other occasions, however, administration of a compound that is at risk of accumulating in milk is desired, for instance in case of administration of a nutrient or a medicament which is beneficial for the health of a human infant or non-human suckling young mammal. In a preferred embodiment it is determined whether a nutrient can be secreted into the milk or colostrum of a lactating animal through active transport by BCRP. If a nutrient appears to be a BCRP substrate, it has a higher chance of being secreted into the milk or colostrum of said lactating animal, as compared to a compound which is not a BCRP substrate. Accumulation in milk is sometimes beneficial to a young mammal or to a milk consuming individual, in which case said nutrient is preferably administered to a lactating animal. As another example, transportation of a medicament into the milk of a lactating animal is desired when a dairy animal suffers from an infection affecting its mammary glands, such as mastitis. If a dairy animal suffers from such disease, administration of a medicament that is primarily transported to mammary gland epithelial cells and the milk is preferred. In this case it is preferred to administer a medicament that is a BCRP substrate.

If secretion of a nutrient and/or medicament into the milk or colostrum is however undesired, one preferably chooses to avoid intake of the nutrient and/or medicament by a lactating animal. Alternatively, or additionally, one chooses to modify said nutrient in order to deprive it from the property of being a BCRP substrate (outlined below in more detail).

In another preferred embodiment a method of the invention is used for testing a medicament, a vaccine, a pesticide, a toxin and/or a carcinogen. These compounds often involve a high risk for young suckling mammals and/or milk consumers. It is therefore important to determine whether these compounds are at risk of accumulating in milk or colostrum. Insight into the risk of accumulation of such compound in milk or colostrum of a lactating mammal helps in determining whether said compound should be administered to a lactating animal (in case said compound is beneficial or if it is not at risk of accumulating in milk or colostrum), or whether administration of said compound to a lactating animal should be avoided and/or whether said compound should be modified in order to deprive it of its property of being secreted into milk or colostrum (if said compound is unfavourable and is at risk of accumulating in milk and/or colostrum). One embodiment thus provides a method of the invention wherein said compound comprises a nutrient, a medicament, a vaccine, a pesticide, a toxin and/or a carcinogen.

A method of the invention is particularly suitable for designing and/or selecting a compound which is not, or to a small extent, secreted into milk and/or colostrum. This is for instance desired when a medicament, vaccine or pesticide is designed and/or selected since a medicament, vaccine or pesticide is beneficial for a lactating animal but often - albeit not always -unfavourable for young suckling mammals and/or milk consumers. Said medicament or vaccine for instance comprises a medicament or vaccine capable of at least treating or preventing an infectious disease. An example of said medicament is an antibiotic. Administration of an antibiotic to a healthy breast-fed infant, healthy non-human suckling animal or a healthy milk consuming individual is often unwanted because it increases the risk of disruption of their gastrointestinal flora, resulting in an increased risk of infection. Moreover, an excess of antibiotics often results in resistance of (pathogenic) micro-organisms.

As another example, a pesticide is often beneficial for a lactating animal but harmful for milk consuming individuals. For instance, said pesticide comprises a composition capable of at least in part counteracting the presence of lice, fleas, insects and/or mites on the skin and/or fur of said animal. Such composition is often applied to the skin/fur of said animal, for instance by spraying. Alternatively said pesticide is administered otherwise such as for instance orally or via injection. A pesticide is often harmful to milk consuming individuals.

Once a compound such as a medicament, vaccine or pesticide has been developed, it is therefore preferably tested with a method of the invention. In a preferred embodiment a plurality of candidate compounds is tested with a method of the invention. When a candidate compound or its metabolite is found not to be a BCRP substrate, it has a smaller chance, as compared to a BCRP substrate, of being transported into the milk of a lactating animal. A compound which is not a substrate of BCRP is therefore preferably used for administration or application to a lactating animal. Using a method of the present invention, a compound such as a medicament, vaccine or pesticide is selected and/or developed that is safely administered or applied to lactating animals such as breast feeding human individuals and dairy animals. In one aspect a method of the invention is therefore provided further comprising selecting a compound which is essentially not a substrate of Breast Cancer Resistance Protein.

If a compound appears to be a BCRP substrate, and if said compound is unfavourable for young suckling mammals or milk consumers, it is advisable not to administer said compound to a lactating mammal. However, said compound is sometimes advantageous, or even indispensable, for a lactating animal. For instance, a lactating animal sometimes needs a medicament which is contra-indicated for young suckling mammals or milk consumers. Moreover, a pesticide such as an anti-lice, anti-flea composition or an insect repellent is often highly advantageous for the well-being of an (adult) animal. If there is no suitable alternative medicament/pesticide available, one often has to provide said lactating animal with said compound, despite its accumulation into milk. This involves a risk for young mammals such as human infants. Moreover, milk of dairy animals which are provided with a harmful compound that accumulates in milk is often not suitable for consumption anymore, resulting in economical loss.

With the teaching of the present invention it has become possible to select and/or design compounds that have a lower risk of accumulating into milk, as compared to BCRP substrates. Now that it is taught by the invention that a compound can accumulate into milk or colostrum through active transport by BCRP, it has become possible to deprive a compound of its property of being secreted into milk or colostrum. This is done by altering said compound such that it is essentially no longer a substrate for BCRP. This is for instance performed by modifying a BCRP binding site of said compound, resulting in impaired transport or, preferably, complete loss of transport by BCRP. A compound is also deprived of its property of being secreted into milk or colostrum by altering its conformation such that a BCRP substrate is no longer capable of binding to BCRP, for instance because of sterical hindrance.

Methods for modifying a compound are widely known in the art. For instance, many methods are available in the art for modifying organic molecules. These methods are commonly known and need no further discussion here. An organic molecule is for instance modified by altering, deleting, substituting and/or adding specific groups such as a hydroxy group or amino group. Of course, a compound is preferably modified such that at least one desired property, such as for instance medical, prophylactic and/or pesticide activity, is maintained, provided or enhanced.
Hence, a harmful compound is preferably altered such that it is essentially not a BCRP substrate anymore, before it is administered to a lactating animal. The invention thus provides a method for at least in part depriving a Breast Cancer Resistance Protein substrate of its property of being secreted into the milk of a lactating mammal if said compound is circulating within said mammal, the method comprising altering said substrate such that the resulting compound is essentially not a Breast Cancer Resistance Protein substrate.

The invention furthermore provides a transgenic non-human mammal wherein an endogenous BCRP gene is operably linked to a heterologous regulatory element which is active in a mammary gland. Preferably, said regulatory element is specific for a mammary gland. A heterologous regulatory element is a sequence which is normally not operably linked to said gene in said animal and which is capable of regulating expression of said gene. Said heterologous regulatory element preferably comprises a promoter and/or an enhancer. A gene and a regulatory element are "operably linked" when said regulatory element is capable of influencing expression of said gene. In one embodiment said regulatory element resides upstream of said gene. In another embodiment however said regulatory element resides downstream of said gene. Said regulatory element is preferably located within 3000 base pairs of said gene. More preferably, said regulatory element is located within 1000 base pairs of said gene, most preferably within 500 base pairs of said gene. A regulatory element is active in a mammary gland when said regulatory element is capable of influencing expression of a gene when present in a mammary gland cell. A regulatory element is specific for a mammary gland if said regulatory element is capable of influencing expression of a gene only when it is present in a mammary gland cell. Said regulatory element is essentially not, or to a significantly lesser extent, capable of influencing expression of a gene when it is present in cell of other tissue.

Methods for generating a transgenic animal are known in the art, for instance described in: Palmiter RD, Brinster RL, Hammer RE, Trumbauer ME, Rosenfeld MG, Birnberg NC, Evans RM. Dramatic growth of mice that develop from eggs microinjected with metallothionein-growth hormone fusion genes. Nature 300:611-615, (1982); and in: Hogan B, Beddington R, Constantini F, Lacy E. Manipulating the mouse embryo, A Laboratory Manual, Cold Spring Harbor Laboratory Press, Cold Spring Harbor, NY, (1994); and in: Krimpenfort P, de Jong R, Uematsu Y, Dembic Z, Ryser S, von Boehmer H, Steinmetz M, Berns A. (1988) Transcription of T cell receptor beta-chain genes is controlled by a downstream regulatory element. EMBO J 7:745-750.

A transgenic non-human animal of the invention is suitable for influencing transport of a BCRP substrate into the milk or colostrum of said animal. For instance, an animal comprising an endogenous BCRP gene operably linked to a heterologous promoter and/or enhancer has an enhanced capability of transporting a BCRP substrate into milk. This is for instance desired if a beneficial compound is collected via the milk of a dairy animal. The invention also allows for at least partial inhibition of secretion of a BCRP substrate into milk or colostrum. This is for instance desired if a harmful compound is administered to said animal. Inhibition of transport of a BCRP substrate is in one embodiment performed by reducing expression of BCRP in the mammary glands, for instance by operably linking a BCRP gene to a repressor. Expression of BCRP in a mammary gland is preferably regulated with an inducible regulatory element such as an inducible promoter, enhancer or repressor. This way, expression of BCRP is temporarily enhanced when a beneficial compound is administered to a lactating animal, and/or temporarily diminished if a harmful compound is administered to a lactating animal. An example of an inducible promoter, enhancer or repressor is the tetracycline repressor-operator system (e.g. Gossen M, Freundlieb S, Bender G, Muller G, Hillen W, Bujard H (1995) Transcriptional activation by tetracyclines in mammalian cells, Science 268:1766-1769).

If enhanced secretion of a BCRP substrate into milk or colostrum is desired, expression of BCRP is preferably enhanced. This is for instance performed by enhancing expression of an endogenous BCRP gene. In one embodiment expression of BCRP is enhanced by providing a non-human animal with a heterologous BCRP gene, or a functional part or analogue thereof, operably linked to a regulatory element which is active in a mammary gland. Preferably, said regulatory element is specific for a mammary gland. This way, a higher amount of BCRP, or functional part, derivative or analogue thereof, in mammary glands is achieved, allowing for an enhanced secretion of a BCRP substrate into milk or colostrum. The invention thus furthermore provides a transgenic non-human mammal comprising a heterologous BCRP gene, or a functional part or analogue of said gene, operably linked to a regulatory element which is specific for a mammary gland.

Said heterologous BCRP or functional part, derivative or analogue thereof preferably has an altered specificity as compared to endogenous BCRP. Said heterologous BCRP or functional part, derivative or analogue thereof is for instance capable of transporting more different beneficial compounds as compared to endogenous BCRP. In one embodiment said heterologous BCRP or functional part, derivative or analogue thereof has a higher affinity for at least one beneficial compound, and/or a lower affinity for at least one adverse compound, as compared to endogenous BCRP.

In another embodiment a non-human animal comprising human BCRP is provided. As outlined above, said animal preferably expresses human BCRP in mammary gland epithelial cells. A non-human animal comprising human BCRP is particularly suitable for determining whether a compound is a substrate of human BCRP. Accumulation of a candidate compound in the milk of said animal indicates that said compound is a substrate of human BCRP, as outlined above. If an animal of the invention is used for this purpose, expression of endogenous BCRP is preferably diminished, more preferably essentially abolished. The invention thus provides a transgenic non-human animal which essentially does not express endogenous BCRP in its mammary gland cells, as well as a use of a non-human animal comprising human BCRP for determining whether a candidate compound is a BCRP substrate.

In one embodiment a candidate compound is administered to a non-human animal comprising (endogenous and/or heterologous) BCRP in its mammary gland cells, and to a non-human animal which essentially does not comprise BCRP in its mammary gland cells. Said animal which essentially does not comprise BCRP preferably comprises a knock out mutant of the same species as said animal comprising BCRP. In one embodiment a wild type mouse and a knock out mouse are used. In another embodiment a mouse, preferably a knock out mouse, provided with human BCRP is used, together with an endogenous BCRP knockout mouse which is not provided with human BCRP. If a candidate compound appears to accumulate in the milk of said animal comprising BCRP, or a functional part, derivative and/or analogue thereof, in its mammary gland cells, while said candidate compound does not, or to a significantly lesser extent, accumulate in the milk of said animal which does not comprise BCRP, or a functional part, derivative and/or analogue thereof, in its mammary gland cells, it indicates that said candidate compound is a BCRP substrate. Moreover, if a second compound is capable of at least in part inhibiting accumulation of said BCRP substrate in the milk of said animal comprising BCRP, or a functional part, derivative and/or analogue thereof, in its mammary gland cells, it indicates that said second compound is a BCRP inhibitor.

When a compound having an adverse effect upon breast-fed infants, young suckling animals and/or milk consumers is administered to a lactating animal, it is preferred to at least in part avoid secretion of said compound into the milk or colostrum of said animal. In one embodiment of the invention a BCRP inhibitor is used for at least in part avoiding secretion of a BCRP substrate into milk or colostrum by BCRP or by a functional part, derivative or analogue of BCRP. A BCRP inhibitor is defined as a compound which is at least in part capable of counteracting transportation of a BCRP substrate by BCRP, and/or by a functional part, derivative and/or analogue of BCRP. BCRP inhibitors are known in the art. Said BCRP inhibitor for instance comprises an organic molecule capable of binding BCRP. More preferably said inhibitor comprises GF120918 (*N*-{4-[2-(1,2,3,4-tetrahydro-6,7-dimethoxy-2-isoquinolinyl)-ethyl]-phenyl}-9,10-dihydro-5-methoxy-9-oxo-4-acridine carboxamide) (Hyafil F et al (1993) Cancer Res.53:4595-4602) or Ko143 (3-(6-Isobutyl-9-methoxy-1,4-dioxo-1,2,3,4,6,7,12,12a-octahydro-pyrazino[1',2':1,6]pyrido[3,4-b]indol-3-yl)-propionic acid tert-butyl ester) (Allen JD et al (2002) *Molecular Cancer Therapeutics.* 1:417-425.), which are small organic molecules. Most preferably said inhibitor comprises GF120918. As is shown in the examples, GF120918 is well capable of inhibiting transport of a BCRP substrate into milk.

Now that it is known that BCRP is capable of transporting BCRP substrates into milk, it has become possible to determine whether a first compound is capable of influencing accumulation of a second compound in milk. If a certain compound is capable of inhibiting BCRP, it is capable of at least in part inhibiting active transport of a BCRP substrate by BCRP, and/or by a functional part, derivative and/or analogue of BCRP. In one embodiment it is therefore determined whether a compound is a BCRP inhibitor. If said compound appears to be a BCRP inhibitor, it is administered to a lactating human individual or animal in order to at least in part prevent a BCRP substrate from accumulating in the milk. For instance, if a medicament, vaccine or pesticide is a BCRP substrate, it is preferably administrated together with a BCRP inhibitor to a lactating human individual or animal in order to at least in part prevent accumulation of said medicament, vaccine or pesticide in milk.

In another embodiment accumulation of a compound such as a nutrient or a medicament in milk is desired. In that case it is preferred to avoid administration of a BCRP inhibitor. Therefore, compounds are preferably screened for BCRP inhibition activity.

The invention thus provides an assay for determining whether a compound is a BCRP inhibitor, characterized in that a mammary gland epithelial cell is used. According to the present invention, mammary gland epithelial cells are suitable for a BCRP inhibition test. A use of a mammary gland epithelial cell for determining whether a compound is a BCRP inhibitor is therefore also provided. One embodiment provides a method for determining whether a candidate compound is an inhibitor of BCRP, comprising determining whether - and if so, to what extent- a BCRP substrate in the presence of said candidate compound is capable of accumulating in milk. Preferably the result of this test is compared with the extent of milk accumulation of said BCRP substrate in the absence of said candidate compound. If less BCRP substrate is capable of accumulating in milk in the presence of said candidate compound as compared to the extent of accumulation of said BCRP substrate in milk in the absence of said candidate compound, it indicates that said candidate compound is a BCRP inhibitor. The invention furthermore provides a use of an assay capable of determining whether a compound is a BCRP inhibitor for determining whether said compound is suitable for co-administration with a second compound to a lactating human individual or animal. If accumulation of a compound in milk is desired, co-administration of a BCRP inhibitor is preferably avoided. However, if accumulation of a compound in milk is undesired, co-administration of a BCRP inhibitor is preferred.

In one embodiment a BCRP inhibitor is separately administered to a lactating animal in order to at least partially inhibit secretion of a BCRP substrate into the milk or colostrum. In another embodiment a BCRP inhibitor and a BCRP substrate are administered at the same time in order to at least in part prevent secretion of said substrate into milk or colostrum. For instance, a mixture of said inhibitor and said BCRP substrate is administered. In yet another embodiment a BCRP inhibitor is coupled to a BCRP substrate. Upon administration of such inhibitor-BCRP substrate molecule, said molecule is cleaved in *vivo* and the resulting inhibitor at least partly prevents secretion of said BCRP substrate into milk or colostrum. The invention thus provides a use of an inhibitor of BCRP for at least in part inhibiting secretion of a BCRP substrate into the milk or colostrum of a lactating mammal.

With the teaching of the invention it has become possible to select a compound which has a lower chance of being secreted into the milk or colostrum of a lactating animal as compared to a BCRP substrate. It is for instance tested whether a candidate compound is a BCRP substrate, allowing for selection of a candidate compound which is not a BCRP substrate. Furthermore, it has become possible to deprive a BCRP substrate of its property of being secreted into milk or colostrum of a lactating animal. This is for instance done by mutating said BCRP substrate and/or by altering the conformation of said BCRP substrate such that the resulting compound is less, or essentially not, capable of being transported by BCRP. A compound selectable or obtainable by a method of the invention which is essentially not a substrate of BCRP is preferably used for preparing a composition which is beneficial for lactating animals but which is unfavourable for suckling young mammals or milk consumers. Said composition for instance comprises a nutrient, medicament, pesticide and/or vaccine. In one embodiment said composition comprises a pesticide. A pesticide which is essentially not a BCRP substrate is preferably used for protecting vegetation that is taken up by lactating dairy animals, since said pesticide does not accumulate in the milk or colostrum of said lactating animals through active transport by BCRP. A use of a compound selected by or obtainable by a method of the invention, for preparing a nutrient, medicament, vaccine and/or pesticide is therefore also herewith provided.

The invention furthermore provides a use of Breast Cancer Resistance Protein, or a functional part, derivative or analogue thereof, for at least in part enhancing secretion of a Breast Cancer Resistance Protein substrate by a mammary gland cell. As outlined above, an increased amount of BCRP, or a functional part, derivative or analogue thereof, in the mammary glands allows for increased secretion of BCRP substrates into milk or colostrum of a lactating animal through active transport by BCRP. In one embodiment said mammary gland cell is cultured ex vivo. A mammary gland cell of the invention preferably comprises a heterologous nucleic acid sequence encoding a Breast Cancer Resistance Protein substrate. Said BCRP substrate is produced by said mammary gland cell and exported by BCRP or by a functional part, derivative or analogue thereof, preferably into the milk or colostrum of a lactating non-human animal or into a culture medium.

With the teaching of the present invention it has become possible to obtain a BCRP substrate. This is in one embodiment performed by providing cells capable of producing a BCRP substrate with BCRP or with a functional part, derivative or analogue thereof. Said cells capable of producing a BCRP substrate are cultured, BCRP substrate is produced by said cells and transported out of the cell by BCRP or by a functional part, derivative or analogue thereof. The invention thus provides a method for obtaining a Breast Cancer Resistance Protein substrate, comprising providing a cell capable of producing said substrate with a Breast Cancer Resistance Protein, or with a functional part, derivative and/or analogue thereof, and culturing said cell under culture conditions that allow the production of said substrate by said cell. Preferably, said substrate is harvested from the culture medium of said cell. Said cells are therefore preferably cultured under conditions that allow extrusion of said substrate by said cell, allowing harvesting of said substrate from the culture medium. In an alternative embodiment said cells are cultured under conditions that are favourable for growth and/or production of said BCRP substrate, but that are less favourable for extrusion and/or harvesting of said BCRP substrate. Said conditions are subsequently changed in order to provide more favourable conditions for extrusion and/or harvesting of said substrate. In this embodiment conditions are adapted to a desired stage of a production process.

A method of the invention is particularly suitable for producing vitamin B2. According to the present invention, vitamin B2 (riboflavin) is a substrate of BCRP. Riboflavin is converted in vivo to the essential coenzymes flavin mononucleotide (FMN) and flavin adenine dinucleotide (FAD), which participate in many key enzymatic redox reactions in the body. Riboflavin is amongst other things needed for growth and for the production of erythrocytes and antibodies. Riboflavin cannot be synthesized by mammals, and there is only limited, short term storage capacity of this vitamin in the liver. Human individuals existing on diets lacking dairy products and meat often have a deficiency of vitamin B2. Therefore, vitamin B2 is usually administered artificially to the diet of these people. Vitamin B2 is currently produced by micro organisms such as yeast. These micro organisms are however not capable of extruding riboflavin into the culture medium. This implicates that the producing cells need to be lysed in order to obtain riboflavin, after which a fresh culture should be started. Moreover, yields are limited because of intracellular inhibition as a result of accumulation of said BCRP in the cells. These limitations are overcome by a method of the invention, since said BCRP substrate is transported out of the cell. Intracellular inhibition is therefore at least partly avoided, resulting in a higher yield of said BCRP substrate. Moreover, the producing cells need not be lysed, so that a culture is maintained.

In one embodiment vitamin B2 is produced by providing a cell capable of producing vitamin B2 with BCRP, or a functional part, derivative and/or analogue thereof, and culturing said cell under culture conditions that allow the production of vitamin B2 by said cell. Preferably, vitamin B2 is harvested from the culture medium of said cell.

Micro-organisms, such as bacteria, fungi or yeast, are especially suitable for use in a method of the invention since micro-organisms are commonly known and cultured. In the art many protocols are available for genetically modifying and culturing micro-organisms and harvesting products produced by said micro-organisms. A preferred embodiment thus provides a method of the invention wherein said cell comprises a cell of a micro-organism, preferably a bacterium, fungus or yeast.

The invention furthermore provides methods for providing a compound with the property of being secreted into the milk or colostrum of a lactating mammal. Secretion of a beneficial compound into milk or colostrum is desired at various occasions. For instance, a medicament or vaccine which is beneficial for a mother is sometimes also beneficial for her baby. For example, it is advantageous that HIV medication received by an HIV infected mother is also transferred to her (possibly) infected baby. However, beneficial compounds such as antiretroviral drugs do not always accumulate in the milk or colostrum of a breastfeeding mother or lactating non-human animal. Therefore, it is preferably determined whether a beneficial compound such as an antiviral drug is a BCRP substrate. A BCRP substrate is preferably selected. According to another embodiment of the present invention such beneficial compounds are provided with a BCRP substrate in order to provide them with the capability of being secreted into the milk or colostrum of a lactating animal through active transport by BCRP. Once a beneficial compound has been provided with a BCRP substrate, it is determined whether the resulting compound is a substrate of BCRP. If a resulting beneficial compound is a BCRP substrate, it is preferably selected and used for administration to a breast feeding mother and/or lactating non-human animal.
The invention thus provides a method for providing a compound with the capability of being secreted into the milk or colostrum of a lactating mammal through active transport by BCRP if said compound is circulating within said mammal, the method comprising providing said compound with a substrate of Breast Cancer Resistance Protein. Said compound preferably comprises a nutrient, a vaccine and/or a medicament, preferably an antiviral drug, since these kinds of compounds are in particular beneficial for young suckling mammals at various occasions. In one embodiment a compound is provided with a BCRP substrate. The invention thus provides a use of a Breast Cancer Resistance Protein substrate for transporting a compound into milk of a lactating mammal. It is also possible to modify a compound such that it becomes a BCRP substrate.

A BCRP substrate selected with a method of the invention, or produced with a method of the invention, is preferably used for preparing a nutrient, medicament and/or a vaccine.

The present invention provides various modifications of a compound. In one embodiment a BCRP substrate is converted into a compound which is not a BCRP substrate, but which is readily metabolized in *vivo* to yield a BCRP substrate. Said compound is converted into a BCRP substrate in *vivo* and transported into the milk of a lactating animal. In a further embodiment, a beneficial compound which is not a BCRP substrate is converted into a prodrug which is a BCRP substrate. Said prodrug is administered to a lactating human individual or non-human animal and subsequently transported into the milk of said human individual or animal. Said prodrug is subsequently converted into said beneficial compound in the milk.

Alternatively, the invention provides means and methods for modifying a compound such that said compound's capability of being a BCRP substrate is altered or essentially lost. A method for altering the capability of a compound of being secreted into the milk of a lactating mammal if said compound is circulating within said mammal, the method comprising altering at least part of said compound such that said compound's capability of being a substrate of Breast Cancer Resistance Protein is altered, is therefore also provided. A method of the invention preferably comprises inducing a modification in said substrate of Breast Cancer Resistance Protein. Alternatively, the structure of said compound is altered such that the BCRP specificity of said compound is altered.

The invention is further explained in the following examples. These examples do not limit the scope of the invention, but merely serve to clarify the invention. Many alternative embodiments can be carried out, which are within the scope of the present invention.

### Examples

### Example 1

### Methods

### Materials

Topotecan and ¹⁴C-topotecan (56 Ci mol⁻¹) were from GlaxoSmithKline (King of Prussia, PA). PhIP and ¹⁴C-PhIP (10 Ci mol⁻¹) were from Toronto Research Chemicals Inc. (Ontario, Canada); ³H-folic acid (26.2 Ci mmol⁻¹) and ¹⁴C-acyclovir (56 Ci mol⁻¹) were from Moravek Biochemicals, Inc. (Brea, CA); ³H-cimetidine (15.5 Ci mmol⁻¹) was from Amersham Life Science (Little Chalfont, UK); ³H-DHEAS (74 Ci mmol⁻¹) was from NEN Life Science Products (Boston, MA); ³H-riboflavin (20 Ci mmol⁻¹), riboflavin, folic acid, cimetidine and DHEAS were from Sigma Chemical Co. (St. Louis, MO).

### Animal experiments

Mice were housed and handled as described (Jonker, J.W., *et al. Proc. Natl. Acad. Sci. USA* 99, 15649-15654 (2002)). For intravenous drug administration, 5 µl drug solution per g body weight was injected into the tail vein of mice lightly anesthetised with methoxyflurane. Levels of radioactivity in plasma and milk were determined by liquid scintillation counting. Measurements of drug secretion into the milk were done in lactating dams with pups of approximately 10 days old. To stimulate milk secretion, oxytocin (200 µl of an 1 I.U. ml⁻¹ solution) was administered subcutaneously to lactating dams at 30 min before milk was collected. After intravenous administration of a drug, at the indicated time approximately 50 µl milk was collected from the 4th and 5th mammary glands by gentle vacuum suction. Immediately after milk collection, a small blood sample was collected from the tail.

### Western analysis and immunohistochemistry

Western blotting and immunohistochemistry were performed as described (Jonker, J.W., *et al. Proc. Natl. Acad. Sci. USA* **99,** 15649-15654 (2002); Maliepaard, M., *et al. Cancer Res.* **61,** 3458-3464 (2001)).
For detection of Bcrp1 and BCRP blots were probed with Mabs BXP-53 (1:20) (Jonker, J.W., *et al. Proc. Natl. Acad. Sci. USA* **99,** 15649-15654 (2002)) and BXP-34 (1:100) (Maliepaard, *M., et al. Cancer Res.* **61,** 3458-3464 (2001)), respectively. For detection of Mrp1, Mrp2 and P-glycoprotein, blots were probed with Mabs MRPr1 (1:1000) (Flens, M.J., *et al. Am. J.Pathol.* **148**, 1237-1247 (1996)), M₂III-5 (1:50) (Scheffer, *G.L., et al. Cancer Res.* **60,** 5269-5277 (2000)) and C219 (1:50; Calbiochem), respectively.

### Vesicular transport assay

Vesicular transport assays were performed as described (Ozvegy, C., *et al.* Functional characterization of the human multidrug transporter, ABCG2, expressed in insect cells. *Biochem. Biophys. Res. Commun.* 285, 111-117 (2001); Zelcer, N., *et al.* Evidence for two interacting ligand-binding sites in human MRP2 (ABCC2). *J. Biol. Chem.* 278, 23538-23544 (2003)), using vesicles from Sf9 cells infected with BCRP-expressing or wild-type baculovirus, in the presence or absence of 4 mM ATP. ATP-dependent transport was calculated by subtracting the uptake in absence of ATP from that in the presence of ATP.

### HPLC analysis

Riboflavin, FMN, and FAD were determined as described (Zempleni, J. Determination of riboflavin and flavocoenzymes in human blood plasma by high-performance liquid chromatography. *Ann. Nutr. Metab.* 39, 224-226 (1995)), with major modifications.
Samples were protected from light during the whole sample pre-treatment. Methanol (4 times the sample volume) was added to plasma and milk samples to precipitate proteins. After mixing, samples were centrifuged 10 min at 10,500 g. Clear supernatants were evaporated to dryness at 40 °C under a stream of nitrogen. Samples were reconstituted in 100 µl of methanol - 50 mM ammonium acetate pH 5 (30:70, v/v) and 25 µl aliquots were injected. The chromatographic system consisted of a Perkin Elmer 200 series pump and ISS 200 autosampler (Perkin-Elmer (PE), Norwalk, CT). Chromatographic separation was performed on a Zorbax SB-C18 column (150 x 4.6 mm ID, 3.5 µm particle size) (Rockland Technologies Inc., Newport, DE). The mobile phase consisted of a mixture of 50 mM ammonium acetate pH 5 and methanol and the following gradient was used: 0-5 min 30% to 90% methanol, 5-7 min 90% methanol, 7-8 min 90% to 30% methanol and 8-12 min 30% methanol. The flow was 1.0 ml min⁻¹ and the detection was performed fluorimetrically using a FP920 Intelligent Fluorescence Detector (Jasco International Co. Ltd., Tokyo, Japan) with excitation/emission wavelengths set at 372/520 nm and 40 nm bandwidth. The capacity of the flow-cell of the fluorescence detector was 16 µl. Riboflavin, FMN, and FAD were eluting after approximately 5.3, 3.9 and 2.5 min, respectively. Processed samples were stable in the autosampler for at least 24 h. Calibration concentrations were between 0.001 and 10 µg ml-1. Calibration curves were calculated by least-squares linear regression using a weighting factor of the reciprocal of the concentration.

### Results

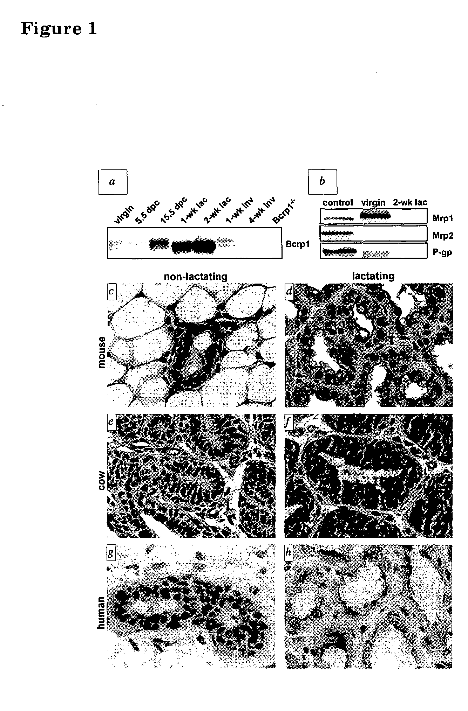

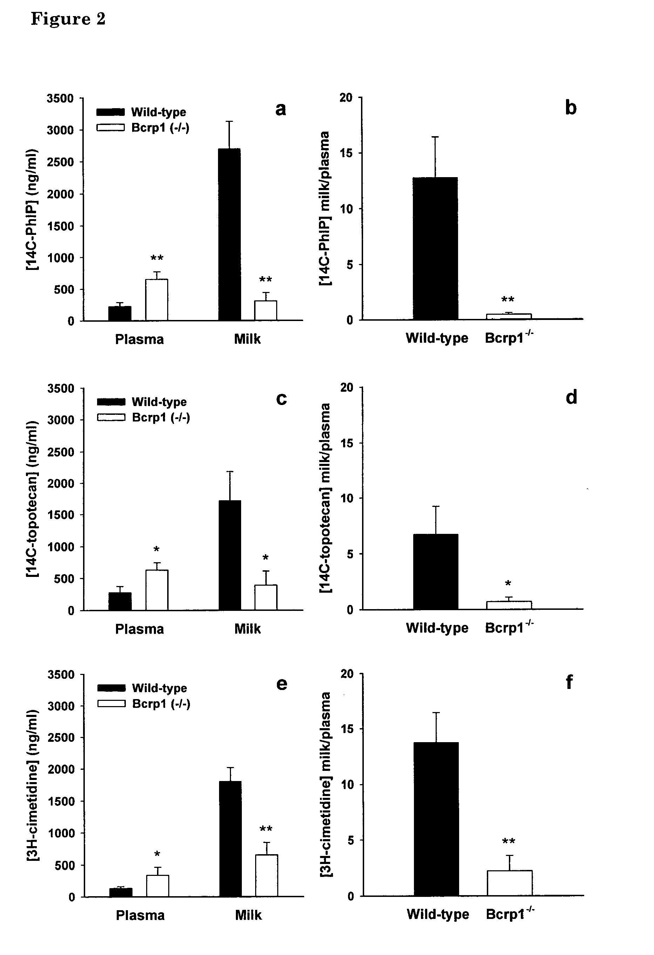

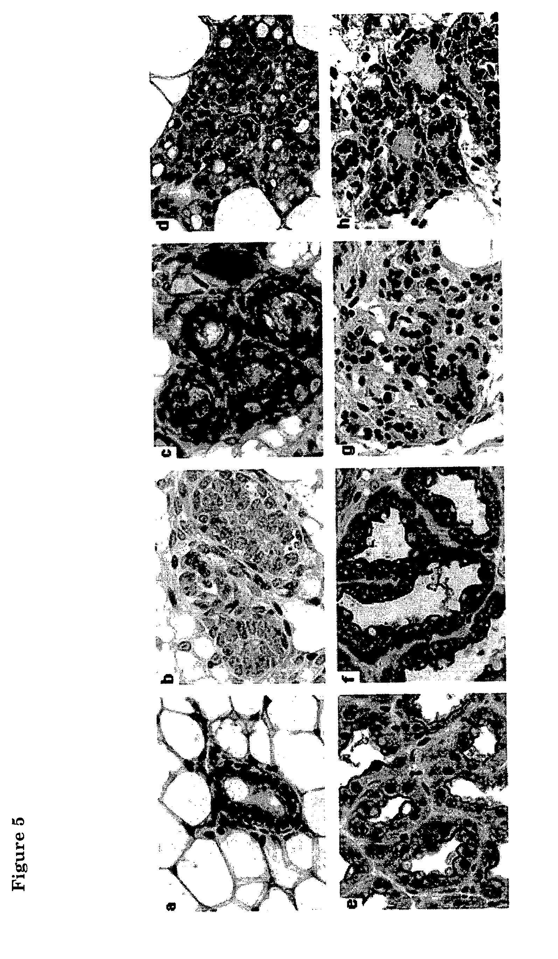

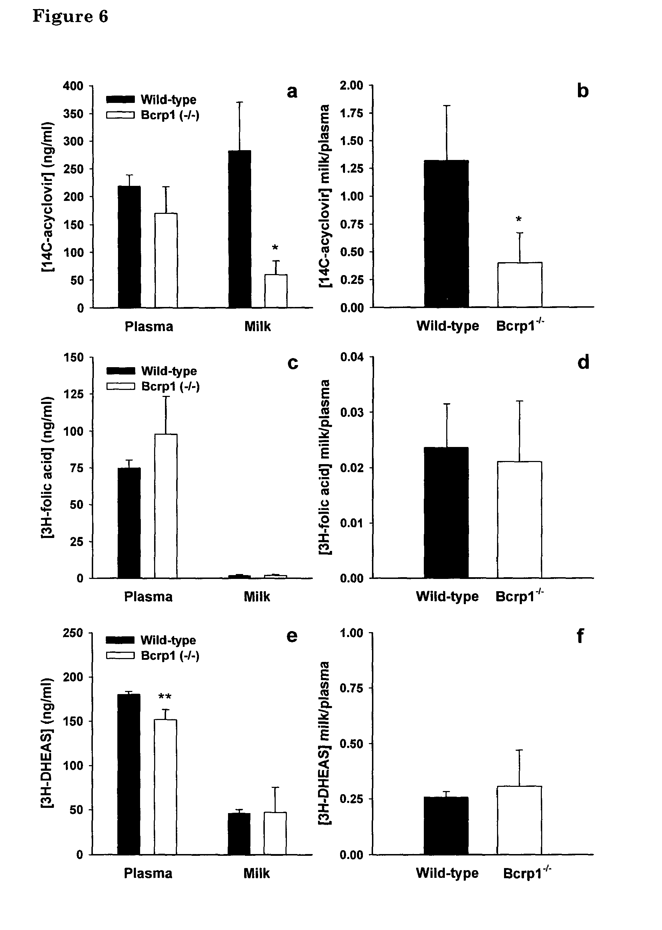

We have demonstrated that Bcrp1, the murine ortholog of BCRP, is highly expressed in mouse mammary gland epithelium during late pregnancy. Analysis of different stages of mammary development by immunohistochemistry and western analysis revealed that Bcrp1 is not expressed in 8 or 14 wk old virgins, but highly induced during late pregnancy and especially lactation (Fig. 1a, c, d, Fig. 5). Bcrp1 was detected primarily in the apical membrane of alveolar epithelial cells, but not in main ducts. During involution following cessation of lactation, expression declined rapidly. We also found high alveolar expression of BCRP in lactating, but not in virgin or nonlactating mammary glands of cows and humans (Fig. 1e-h).
To examine the functional role of Bcrp1 in the mammary gland, we compared the milk secretion of the known Bcrp1 substrates PhIP and topotecan after intravenous administration to lactating wild-type and *Bcrp1-*/*-* mice (Van Herwaarden, A.E., *et al. Cancer Res.* 63, 6447-6452 (2003); Jonker, J.W., *et al. J. Natl. Cancer. Inst.* 92, 1651-1656 (2000); Jonker, J.W., *et al. Proc. Natl. Acad. Sci. USA* 99, 15649-15654 (2002)) (Fig. 2a-d).
Whereas in wild-type mice both compounds were highly concentrated into the milk, as indicated by high milk-to-plasma (M/P) ratios, active milk secretion of both compounds was abolished in the *Bcrp1-*/*-* mice (M/P <= 1). Note that plasma levels of intravenously administered Bcrp1 substrates are generally higher in *Bcrp1-*/*-* mice due to decreased elimination. Interestingly, we have identified several novel Bcrp1 substrates merely based on previously published high M/P ratios, for example the anti-ulcerative cimetidine, the antiviral drug acyclovir (Fig. 2e-f, Fig. 6a-b), as well as a range of other drugs and carcinogens (to be described elsewhere). Other broad-specificity apical multidrug transporters, P-glycoprotein (Abcb1a/b) and Mrp2 (Abcc2), are absent from lactating breast, as is Mrp1 (Abcc1) (Fig. 1b). Our data thus show that BCRP/Bcrp1 is responsible for the concentrative transfer of drugs, carcinogens and dietary toxins to the milk of mammals such as mice, cows and humans.

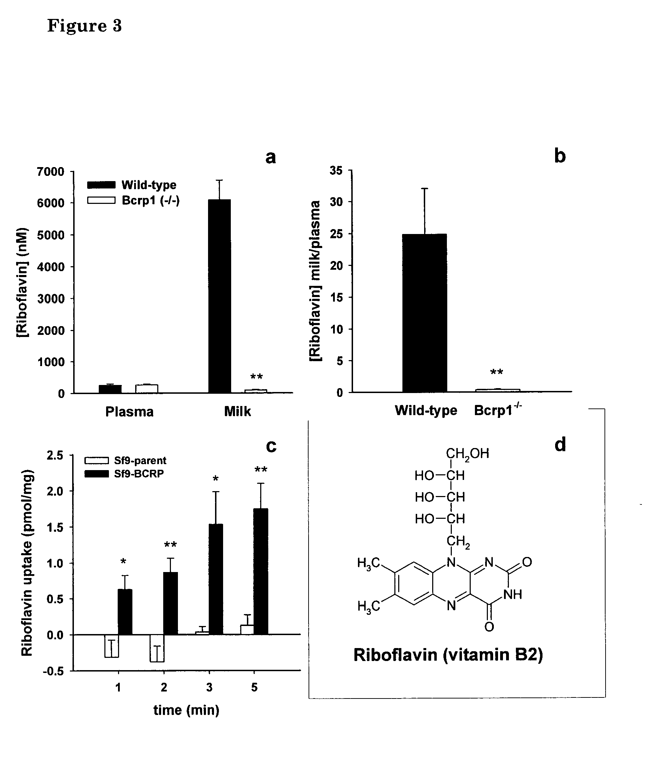

We furthermore tested a number of vitamins and other compounds known to be concentrated into milk, and structurally resembling BCRP substrates. While some obvious candidates (e.g., the BCRP substrates folic acid and dehydroepiandrosterone-sulfate (DHEAS) (Fig. 6c-f), and the porphyrin vitamin B12 (not shown) were not affected, we observed a dramatic, 67-fold decrease in the endogenous milk secretion of vitamin B2 (riboflavin) in *Bcrp1* -/- compared to wild-type mice (Fig. 3a-b, d). The riboflavin M/P ratio dropped from 24.8 ± 7.2 in wild-type to 0.37 ± 0.11 in *Bcrp1-*/*-* mice, demonstrating the strong concentrative action of Bcrp1. Subsequent in vitro inside out vesicle uptake experiments confirmed ATP-dependent transport of riboflavin by human BCRP (Fig. 3c). BCRP/Bcrp1 is thus responsible for pumping vitamin B2 into milk. Importantly, milk is a primary source of riboflavin and a deficiency is often endemic in human populations that exist on diets lacking dairy products and meat.

This study identifies for the first time a molecular mechanism responsible for concentrating a vitamin into milk. Surprisingly, rather than evolving a dedicated transporter, the mammary gland has recruited a broad-specificity multidrug transporter to pump vitamin B2 into milk. As an inevitable consequence, a huge range of dietary toxins and carcinogens, but also drugs and pesticides and their metabolites, is liable to be concentrated into milk.

### Example 2

### Inhibition of Bcrp1-mediated milk secretion of topotecan by GF120918

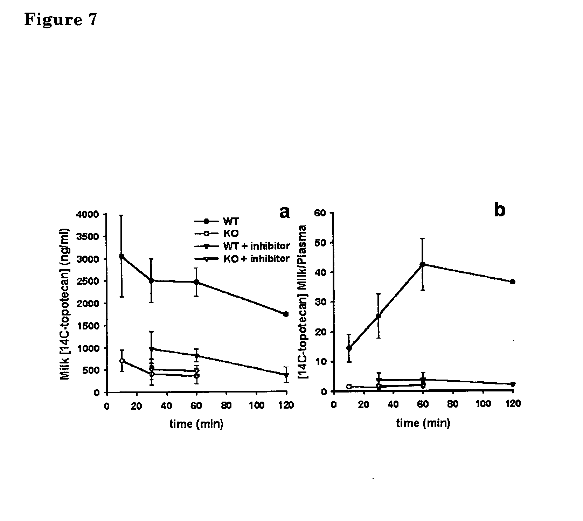

Active secretion of topotecan into the milk by Bcrp1 could be effectively reversed by oral administration of the Bcrp1-inhibitor GF120918 to mice, demonstrating the feasibility of reducing milk contamination with Bcrp1 substrates (Figure 7).

### Methods

Milk secretion experiments were done in lactating dams with pups of approximately 10 days old. GF120918 was suspended at 5 mg/ml in water for injection and concentrated stock vehicle (1:1). The concentrated stock vehicle consisted of hydroxypropyl methyl cellulose (10 g/l) and 2% Tween 80 in water for injection. Animals were administered GF120918 (250 mg/kg; 10 µl of drug solution/g body weight) by gavage into the stomach at 2 hours before [¹⁴C]-topotecan injection. [¹⁴C]-topotecan (1 mg/kg) was injected into the vein of mice lightly anesthetized with methoxyflurane. To stimulate milk secretion, oxytocin (200 µl of 1 I.U./ml solution) was administered subcutaneously to lactating dams at 15 min before each milk sample was collected. At indicated time points (15, 30, 60 and 120 min after [¹⁴C]-topotecan injection), 50 µl of milk was collected from the mammary glands by gentle vacuum suction. Immediately after milk collection, a small blood sample was collected from the tail. At the last sampling point, animals were sacrificed by cardiac puncture after anesthesia with methoxyflurane.
Figure 7 shows milk [¹⁴C] topotecan concentration and M/P ratio versus time curves in wild-type and Bcrp1 -knockout mice, with or without Bcrp 1-inhibitor GF120918 (250 mg/kg orally at T=-2 hours). It is shown that GF120918 is capable of at least in part inhibiting transport of topotecan into the milk.

### Brief description of the drawings

Figure 1. Expression of Bcrp1/BCRP in the mammary gland. a, Western analysis on crude membrane fractions from mouse mammary glands at different stages (10 µg protein per lane).
   b, Western analysis of Mrp1, Mrp2, and P-glycoprotein in mammary glands of virgins and 2-wk lactating female wild-type mice. c-h, Immunohistochemical detection (×100) in (c) 14-wk old virgin mouse, (d) 1-wk lactating mouse, (e) virgin cow, (f) lactating cow, (g) nonlactating human, (h) lactating human.
**Figure 2.** Bcrp1 mediates active secretion of compounds into the milk. a-f, Radiolabelled compounds (1 mg kg-1) were intravenously administered to lactating wild-type or *Bcrp1-*/*-* females and after 30 min, plasma and milk concentrations and milk/plasma (M/P) ratios were determined for (a-b) ¹⁴C-PhIP, (c-d) ¹⁴C-topotecan, and (e-f) ³H-cimetidine. Data represent the mean ± s.d., n = 3-4, **P*< 0.05, ***P*< 0.01 (two-tailed unpaired Student's t-test).
**Figure 3.** Bcrp1 transports riboflavin (vitamin B2) and mediates its active secretion into milk.
   a, b, Endogenous riboflavin concentrations in (a) plasma and milk of wild-type and *Bcrp1-l-* mice, and (b) M/P ratios in lactating dams; Data represent the mean ± s.d., n = 3-4, **P < 0.01 (two-tailed unpaired Student's t-test). c, BCRPmediated uptake of ³H-riboflavin (0.25 µM) in membrane vesicles from Sf9 cells infected with BCRP-expressing or wild-type baculovirus; Data represent the mean uptake ± s.e. in the presence of ATP minus the uptake in the absence of ATP, *n* = 6, **P*< 0.05, ***P*< 0.01, comparing differences between uptake in parent and BCRP vesicles. d, Chemical structure of riboflavin.

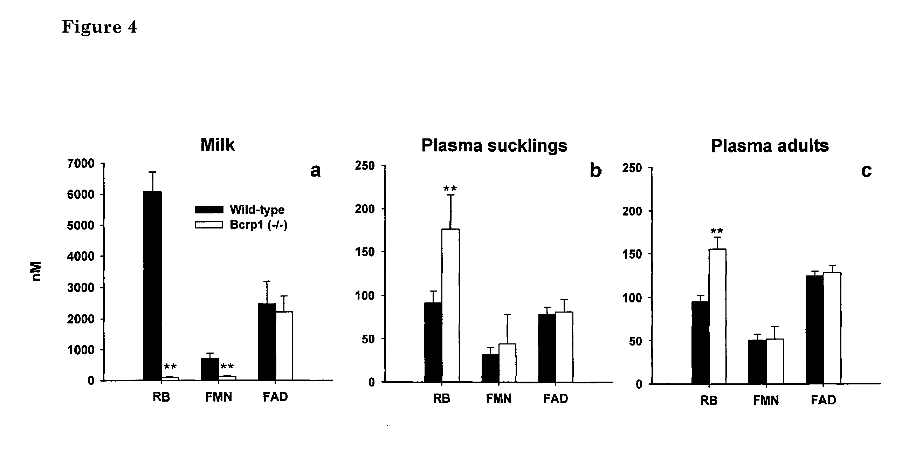

Figure 4. Endogenous levels of riboflavin (RB), and its coenzyme forms FMN and FAD in (a) milk of wild-type and *Bcrpl-*/*-* mice (n = 3-4), (b) plasma of 10-day old sucklings (n = 9), and (c) plasma of adult males (n = 6-9). Data represent the mean ± s.d., **P < 0.01 (two-tailed unpaired Student's t-test).
Figure 5. Expression of Bcrp1 during mammary development. a-h, Immunohistochemical detection (×100) in wild-type mice (a) 14-wk old virgin, (b) 5.5 days post coitus (dpc), (c) 15.5 dpc, (d) negative control *(Bcrp1-*/*-,* 15.5 dpc), (e) 1-wk lactating, (f) 2-wk lactating, (g) 1-wk involution, (h) 4-wk involution.
**Figure 6.** Milk secretion of acyclovir, folic acid and DHEAS in mice. a-f, Radiolabelled compounds (1 mg kg⁻¹) were intravenously administered to lactating wild-type or *Bcrp1-*/*-* females and after 30 min, plasma and milk concentrations and milk/plasma (M/P) ratios were determined for (a-b) ¹⁴C-acyclovir, (c-d) ³H-folic acid, and (e-f) ³H-DHEAS. Data represent the mean ± s.d., n = 3-4, **P* < 0.05, ***P* < 0.01 (two-tailed unpaired Student's ttest).
**Figure 7.** Milk [¹⁴C] topotecan concentration- (a) and M/P ratio- (b) versus time curves in wild-type and Bcrp1 -knockout mice, with or without Bcrp1-inhibitor GF120918 (250 mg/kg orally at T=-2 hours). Data represent the mean ± sd., n=3-6 (n=1 for wild-type at T= 120 min).

### References

Allen JD, van Loevezijn A, Lakhai JM, van der Valk M, van Tellingen O, Reid G, Schellens JHM, Koomen G-J, Schinkel AH (2002) Potent and specific inhibition of the Breast Cancer Resistance Protein multidrug transporter in *vitro* and in mouse intestine by a novel analogue of Fumitremorgin C. *Molecular Cancer Therapeutics.* 1:417-425
Allen JD, Schinkel AH (2002) Multidrug resistance and pharmacological protection mediated by the Breast Cancer Resistance Protein (BCRP/ABCG2). *Molecular Cancer Therapeutics* Vol. 1: 427-434
Flens, M.J., *et al.* Tissue distribution of the multidrug resistance protein. *Am. J. Pathol.* **148,** 1237-1247 (1996).
Gossen M, Freundlieb S, Bender G, Muller G, Hillen W, Bujard H (1995) Transcriptional activation by tetracyclines in mammalian cells, Science 268:1766-1769
Hogan B, Beddington R, Constantini F, Lacy E. Manipulating the mouse embryo, A Laboratory Manual, Cold Spring Harbor Laboratory Press, Cold Spring Harbor, NY, (1994); and in: Krimpenfort P, de Jong R, Uematsu Y, Dembic Z, Ryser S, von Boehmer H, Steinmetz M, Berns A. (1988) Transcription of T cell receptor beta-chain genes is controlled by a downstream regulatory element. EMBO J 7:745-750
Hyafil F, Vergely C, Du Vignaud P, Grand-Perret T (1993) In vitro and in vivo reversal of multidrug resistance by GF120918, an acridonecarboxamide derivative. Cancer Res.53:4595-4602
Jonker, J.W., *et al.* Role of breast cancer resistance protein in the bioavailability and fetal penetration of topotecan. J. *Natl. Cancer. Inst.* 92, 1651-1656 (2000).
Jonker, J.W., *et al.* The breast cancer resistance protein protects against a major chlorophyll-derived dietary phototoxin and protoporphyria. *Proc. Natl. Acad. Sci. USA* **99,** 15649-15654 (2002).
Maliepaard, M., *et al.* Subcellular localization and distribution of the breast cancer resistance protein transporter in normal human tissues. *Cancer Res.* 61, 3458-3464 (2001).
Ozvegy, C., *et al.* Functional characterization of the human multidrug transporter, ABCG2, expressed in insect cells. *Biochem. Biophys. Res. Commun.* 285, 111-117 (2001).
Palmiter RD, Brinster RL, Hammer RE, Trumbauer ME, Rosenfeld MG, Birnberg NC, Evans RM. Dramatic growth of mice that develop from eggs microinjected with metallothionein-growth hormone fusion genes. Nature 300:611-615, (1982)
Scheffer, G.L., *et al.* Specific detection of multidrug resistance proteins MRP1, MRP2, MRP3, MRP5, and MDR3 P-glycoprotein with a panel of monoclonal antibodies. *Cancer Res.* 60, 5269-5277 (2000).
Van Herwaarden, A.E., *et al.* The Breast Cancer Resistance Protein (Bcrp1/Abcg2) restricts exposure to the dietary carcinogen 2-amino-1-methyl-6-phenylimidazo[4,5-b]pyridine (PhIP). *Cancer Res.* 63, 6447-6452 (2003).
Zelcer, N., *et al.* Evidence for two interacting ligand-binding sites in human MRP2 (ABCC2). *J. Biol. Chem.* **278**, 23538-23544 (2003).
Zempleni, J. Determination of riboflavin and flavocoenzymes in human blood plasma by high-performance liquid chromatography. *Ann. Nutr. Metab.* **39,** 224-226 (1995).

## Claims

1. A method for determining whether a compound or its metabolite can be transported through active transport by Breast Cancer Resistance Protein into the milk or colostrum of a lactating mammal if said compound or metabolite is circulating within said mammal, the method comprising determining whether said compound or metabolite is a substrate of Breast Cancer Resistance Protein.

2. A method according to claim 1, wherein said compound comprises a nutrient, a medicament, a vaccine, a pesticide, a toxin and/or a carcinogen.

3. A method according to claim 1 or 2, wherein said mammal comprises a human or a dairy mammal.

4. A method according to any one of claims 1-3 further comprising selecting a compound which is essentially not a substrate of Breast Cancer Resistance Protein.

5. A method according to any one of claims 1-4 further comprising selecting a compound which is a substrate of Breast Cancer Resistance Protein.

6. A method according to any one of claims 1-5, comprising determining in vitro whether said compound or metabolite is a substrate of Breast Cancer Resistance Protein

7. A method for at least in part depriving a Breast Cancer Resistance Protein substrate of its property of being secreted into the milk of a lactating mammal if said compound is circulating within said mammal, the method comprising altering said substrate such that the resulting compound is essentially not a Breast Cancer Resistance Protein substrate.

8. A transgenic non-human mammal wherein an endogenous BCRP gene is operably linked to a heterologous regulatory element which is specific for a mammary gland.

9. A transgenic non-human mammal comprising a heterologous BCRP gene or a functional part or analogue thereof operably linked to a regulatory element which is specific for a mammary gland.

10. A transgenic non-human animal whose endogenous BCRP gene is at least in part deleted and/or silenced.

11. Use of a non-human animal according to claim 9 or 10 for determining whether a candidate compound is a BCRP substrate.

12. Use of an inhibitor of BCRP for at least in part inhibiting secretion of a BCRP substrate into the milk or colostrum of a lactating mammal.

13. Use according to claim 12, wherein said inhibitor comprises GF120918.

14. Use of a compound selected with a method according to claim 4 or obtainable by a method according to claim 7 for preparing a nutrient, medicament, vaccine and/or pesticide.

15. Use of Breast Cancer Resistance Protein for at least in part enhancing secretion of a Breast Cancer Resistance Protein substrate by a mammary gland cell.

16. Use according to claim 15, wherein said cell is cultured ex *vivo.*

17. Use according to claim 15 or 16, wherein said cell comprises a heterologous nucleic acid sequence encoding a Breast Cancer Resistance Protein substrate.

18. A method for obtaining a Breast Cancer Resistance Protein substrate, comprising providing a cell capable of producing said substrate with a Breast Cancer Resistance Protein, or a functional part, derivative and/or analogue thereof, and culturing said cell under culture conditions that allow the production of said substrate by said cell.

19. A method according to claim 18, further comprising harvesting said substrate from the culture medium of said cell.

20. A method according to claim 18 or 19, wherein said Breast Cancer Resistance Protein substrate comprises vitamin B2.

21. A method according to any one of claims 18-20, wherein said cell comprises a cell of a micro-organism, preferably a bacterium, fungi or yeast.

22. A method for providing a compound with the property of being secreted into the milk or colostrum of a lactating mammal if said compound is circulating within said mammal, the method comprising providing said compound with a substrate of Breast Cancer Resistance Protein and determining whether the resulting compound is a substrate of Breast Cancer Resistance Protein.

23. A method according to claim 22, wherein said compound comprises a nutrient, a vaccine and/or a drug, preferably an antiviral drug.

24. A method for altering the capability of a compound of being secreted into the milk of a lactating mammal if said compound is circulating within said mammal, the method comprising altering at least part of said compound such that said compound's capability of being a substrate of Breast Cancer Resistance Protein is altered.

25. A method according to claim 24, comprising inducing a modification in said substrate of Breast Cancer Resistance Protein.

26. Use of an assay for determining whether a compound is a substrate of Breast Cancer Resistance Protein, for determining whether a compound can be transported into the milk or colostrum of a lactating mammal through active transport by Breast Cancer Resistance Protein if said compound is circulating within said mammal.

27. Use of a Breast Cancer Resistance Protein substrate for transporting a compound into milk of a lactating mammal.

28. Use of a compound selected with a method according to claim 5 or obtainable by a method according to any one of claims 18-22 for preparing a nutrient, medicament and/or vaccine.
